## Europäisches Patentamt

## European Patent Office

## Office européen des brevets

(11) Veröffentlichungsnummer : **0 182 333**
**B1**

(12) **EUROPÄISCHE PATENTSCHRIFT**

(45) Veröffentlichungstag der Patentschrift :
02.03.88

(51) Int. Cl.⁴ : **C 07 C   6/06, C 07 C 13/02**

(21) Anmeldenummer : 85114626.6

(22) Anmeldetag : 18.11.85

(54) **Verfahren zur Herstellung von Cycloalkadienen.**

(30) Priorität : **20.11.84 DE 3442376**
**12.07.85 DE 3524977**

(43) Veröffentlichungstag der Anmeldung :
**28.05.86 Patentblatt 86/22**

(45) Bekanntmachung des Hinweises auf die Patenterteilung : **02.03.88 Patentblatt 88/09**

(84) Benannte Vertragsstaaten :
**AT BE CH DE FR GB IT LI NL**

(56) Entgegenhaltungen :
**GB-A- 1 105 565**
**GB-A- 1 118 517**
**GB-A- 2 064 354**

(73) Patentinhaber : **Consortium für elektrochemische Industrie GmbH**
**Zielstattstrasse 20**
**D-8000 München 70 (DE)**

(72) Erfinder : **Eberle, Hans-Jürgen. Dr. Dipl.-Chem.**
**Höglwörther Strasse 351**
**D-8000 München 70 (DE)**
Erfinder : **Kreuzer, Franz-Heinrich, Dr. Dipl.-Chem.**
**Josef-Gerstner-Strasse 14**
**D-8033 Martinsried (DE)**
Erfinder : **Zeitler, Norbert**
**Ganghoferstrasse 21**
**D-8000 München 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Cycloalkadienen in Flüssigphase durch Metathesereaktion von Cycloalkenen in Gegenwart eines Trägerkatalysators auf Basis $Re_2O_7/Al_2O_3$.

Es ist gemäß GB 1 105 565 bereits bekannt, Cyclohexadekadien durch Metathesereaktion von Cycloocten an einem $Re_2O_7/Al_2O_3$Kontakt herzustellen. Dabei wird bevorzugt nach Art einer Feststoffextraktion in einer Soxhlet-Apparatur gearbeitet. Die Ausbeute wird mit 6 %, bezogen auf die Menge eingesetzten Cyclooctens, angegeben.

Der dem genannten Verfahren zugrundeliegende Reaktionstyp kann als Dimerisierung des Ausgangsprodukts beschrieben werden. Die unbefriedigende Ausbeute ist nicht zuletzt darauf zurückzuführen, daß die Reaktion mit ungenügender Selektivität in Bezug auf die Dimerisierung verläuft und zum großen Teil höhere Oligomere gebildet werden.

Es wurde nun gefunden, daß bei der Metathesereaktion von Cycloalkenen das Stadium der Dimerisierung, bzw. für den Fall, daß unterschiedliche Ausgangsprodukte eingesetzt werden, ein dem der Dimerisierung äquivalentes Stadium, in weit geringerem Maße überschritten wird wenn mit hochverdünnten Lösungen der Ausgangsprodukte gearbeitet wird.

Gegenstand der Erfindung ist ein Verfahren zur Herstellung von Cycloalkadienen in Flüssigphase durch Metathesereaktion von Cycloalkenen in Gegenwart eines Trägerkatalysators auf Basis $Re_2O_7/Al_2O_3$, das dadurch gekennzeichnet ist, daß die Cycloalkene als 0,01 bis 0,05 molare Lösungen bei Verweilzeiten von 10 bis 100 sec mit dem Tragerkatalysator in Kontakt gebracht werden.

Das erfindungsgemäße Verfahren ist insbesondere zugeschnitten zur Herstellung von Cyclotetradekadien-1.8 und Cyclohexadekadien-1.9, die durch Dimerisieren von Cyclohepten bzw. Cycloocten zugänglich sind, sowie von Cyclopentadekadien-1.8 und Cycloheptadekadien-1.6, die durch Metathese von Cyclohepten und Cyclooocten bzw. Cyclopenten und Cyclododecen gewonnen werden können.

Es werden insbesondere solche Trägerkatalysatoren eingesetzt, die $\gamma$-$Al_2O_3$ als Trägermaterial aufweisen. Die spezifische Oberfläche des Trägermaterials beträgt zweckmäßigerweise 100 bis 300 $m^2/g$ nach BET.

Das Trägermaterial wird insbesondere in Form von Hohlsträngen, Kugeln, Zylindern, Würfeln oder Kegeln eingesetzt.

Der Gewichtsanteil an $Re_2O_7$ am Gesamtgewicht des Katalysators beträgt vorzugsweise 3 bis 20 Gew.-%, insbesondere 3 bis 7 Gew.-%.

Es ist im Rahmen der Erfindung bevorzugt, solche Trägerkatalysatoren auf Basis $Re_2O_7/\gamma$-$Al_2O_3$ einzusetzen, die noch mit Zinntetraalkylen als Co-Katalysatoren beladen sind. Die Menge an Co-Katalysator wird so bemessen, daß das molare

Verhältnis von $Re_2O_7$ zu Co-Katalysator 5 : 1 bis 1 : 1 beträgt.

Beispiele für Co-Katalysatoren sind Zinntetramethyl, Zinntetraethyl, Zinn-tetra-n-butyl.

Die Herstellung der erfindungsgemäß einzusetzenden Trägerkatalysatoren ist an sich bekannt, sie erfolgt beispielsweise durch Imprägnieren des Trägermaterials mit einer wäßrigen Lösung von Ammoniumperrhenat mit anschließender thermischer Behandlung des Guts, wobei die Rheniumverbindung in das Oxid übergeführt wird.

Die Beladung des $Re_2O_7/\gamma$-$Al_2O_3$-Kontakts mit Co-Katalysator erfolgt zweckmäßigerweise durch Behandeln des Katalysatormaterials mit Lösungen der entsprechenden Zinntetraalkyle in aliphatischen oder aromatischen Kohlenwasserstoffen. Beispiele für derartige Lösungsmittel sind Metathese-inerte Lösungsmittel, die auch zur Verdünnung der Ausgangssubstanzen eingesetzt werden, wie beispielsweise Pentan, Hexan, Heptan, Cyclopentan, Cyclohexan, Petrolether, Methylenchlorid, Chloroform, Tetrachlorkohlenstoff, Chlorbenzol, m-Dichlorbenzol.

Die erfindungsgemäß der Metathesereaktion zu unterwerfenden Cycloolefine werden als 0,01 bis 0,05, vorzugsweise 0,01 bis 0,03 molare Lösungen in Metathese-inerten Lösungsmitteln eingesetzt. Für den Fall der Co-Metathese, wobei verschiedene Cycloalkene als Ausgangssubstanzen eingesetzt werden, beziehen sich die Konzentrationsangaben auf die Summe der Ausgangssubstanzen. Die Ausgangssubstanzen werden dabei im Molverhältnis 1 : 1 oder in etwa 1 : 1 eingesetzt.

Beispiele für Metathese-inerte Lösungsmittel sind insbesondere Pentan, Hexan, Heptan, Cyclopentan, Cyclohexan, Petrolether vom Siedebereich 30 bis 60 °C, Methylenchlorid, Chloroform, Tetrachlorkohlenstoff.

Erfindungsgemäß wird in einer Festbettanordnung gearbeitet, wobei gelöstes Cycloalken das Katalysatorbett als Flüssigphase durchströmt. Die Strömungsgeschwindigkeit wird erfindungsgemäß dabei so eingestellt, daß Verweilzeiten von 10 bis 100 sec eingehalten werden.

Die Reaktionstemperaturen liegen in der Regel im Bereich von 0 bis 50 °C.

Beispielsweise wird zur Durchführung des Verfahrens ein mit Katalysator gefüllter Rohrreaktor in vertikaler Anordnung von einer Lösung von Cycloalken von oben nach unten durchströmt. Das den Reaktor verlassende Reaktionsgemisch wird einer Destillationsvorrichtung aufgegeben und in seine Komponenten getrennt. Das Zielprodukt fällt als relativ hochsiedende Fraktion an. In der Praxis wird zumeist ein kontinuierlicher Betrieb aufrechterhalten, wobei das als Leichtsieder anfallende Lösungsmittel sowie ggf. nicht umgesetztes Ausgangsprodukt im Kreislauf geführt und nach Beschickung mit neuem Ausgangsprodukt in den Reaktor zurückgeleitet wird.

Nach dem erfindungsgemäßen Verfahren gelingt es, makrocyclische Alkadiene mit 14 bis 17 Kohlenstoffatomen in guter Ausbeute herzustellen. Die Selektivität der Reaktion liegt, bezogen auf die Menge eingesetzten Cycloalkens bei 35 bis 50 %. Es gelingt damit, chemisch an sich schwer zugängliche Verbindungen in wirtschaftlicher Weise herzustellen. Die Verfahrensprodukte finden insbesondere Verwendung auf dem Riechstoffsektor, beispielsweise als Ausgangsprodukte für die Herstellung von Moschus-Riechstoffen.

Die Erfindung wird nun anhand von Beispielen näher erläutert :

Beispiel 1

Herstellung von Cyclohexadecadien-1.9

Es wurde ein Rohrreaktor (Länge 70 cm, Durchmesser 8 cm) in vertikaler Anordnung verwendet, der mit 1,8 kg Trägerkatalysator beaufschlagt war. Der Katalysator enthielt 3,5 Gew.-% $Re_2O_7$ und 1,3 Gew.-% Zinntetramethyl. Als Trägermaterial wurde ein zu Hohlsträngen geformtes γ-Aluminiumoxid verwendet mit einer spezifischen Oberfläche von 190 $m^2$/g. Durch das Katalysatorbett wurde eine 0,018 molare Lösung von Cyclooocten in n-Pentan geleitet. Die Verweilzeit des Gemisches betrug 60 sec. Die Temperatur im Katalysatorbett wurde auf 15 °C eingestellt.

Das den Reaktor verlassende Reaktionsgemisch wurde einer Destillationsvorrichtung zugeführt, wobei n-Pentan abdestilliert und nach Beladen mit frischem Cyclooocten wieder in den Reaktor zurückgeführt wurde.

Die destillative Auftrennung ergab schließlich in einer Ausbeute von 33,5 % der Theorie, bezogen auf eingesetztes Cyclooocten, Cyclohexadecadien-1.9 Die Selektivität der Reaktion betrug 37 %.

Beispiel 2

Herstellung von Cyclopentadekadien-1.8

Es wurde die Arbeitsweise gemäß Beispiel 1 wiederholt, mit der Abänderung, daß anstatt einer 0,018 molaren Lösung von Cyclooocten in n-Pentan ein Cyclooocten/Cyclohepten-Gemisch im molaren Verhältnis 1 : 1,1 in n-Pentan eingesetzt wurde, wobei die Gesamtkonzentration an Cycloalken 0,019 Mol/l betrug. Es wurde eine Verweilzeit des Gemisches im Katalysatorbett von 70 sec eingehalten.

Die destillative Auftrennung des Reaktionsgemisches ergab eine Ausbeute von
    10,5 % Cyclotetradekadien-1.8
    10,9 % Cyclopentadekadien-1.8 und
    15,3 % Cyclohexadekadien-1.9

**Patentanspruch**

Verfahren zur Herstellung von Cycloalkadienen in Flüssigphase durch Metathese-Reaktion von Cycloalkenen in Gegenwart eines Trägerkatalysators auf Basis $Re_2O_7/Al_2O_3$, dadurch gekennzeichnet, daß die Cycloalkene als 0,01 bis 0,05 molare Lösungen bei Verweilzeiten von 10 bis 100 sec mit dem Trägerkatalysator in Kontakt gebracht werden.

**Claim**

Process for the preparation of cycloalkadienes in the liquid phase by a metathesis reaction of cycloalkenes in the presence of a supported catalyst based on $Re_2O_7/Al_2O_3$, characterized in that the cycloalkenes are contacted as 0.01 to 0.05 molar solutions with the supported catalyst at residence times from 10 to 100 seconds.

**Revendication**

Procédé pour préparer des cycloalcadiènes en phase liquide par une réaction de métathèse sur des cycloalcènes, en présence d'un catalyseur sur support à base de $Re_2O_7/Al_2O_3$, procédé caractérisé en ce que les cyclo-alcènes, sous la forme de solutions d'une molarité comprise entre 0,01 et 0,05, sont mis en contact avec le catalyseur sur support pendant des temps de séjour de 10 à 100 secondes.